# EUROPEAN PATENT SPECIFICATION

(11) **EP 3 831 933 B1**
(45) Date of publication and mention of the grant of the patent: **29.10.2025**
(21) Application number: 19842104.2
(22) Date of filing: 26.07.2019
(51) Int. Cl.: C12N 5/071, C12N 5/0735

(54) **METHOD FOR PRODUCING INTESTINAL EPITHELIAL CELLS AND INTESTINAL EPITHELIAL CELLS**
VERFAHREN ZUR HERSTELLUNG VON INTESTINALEN EPITHELZELLEN UND INTESTINALE EPITHELZELLEN
PROCÉDÉ DE PRODUCTION DE CELLULES ÉPITHÉLIALES INTESTINALES ET CELLULES ÉPITHÉLIALES INTESTINALES

(30) Priority: 27.07.2018 JP 2018141703
(43) Date of publication of application: 09.06.2021
(73) Proprietor: FUJIFILM Corporation, Tokyo 106-8620 (JP)
(72) Inventor: MIMA Shinji, Ashigarakami-gun, Kanagawa 258-8577 (JP); IMAKURA Yuki, Ashigarakami-gun, Kanagawa 258-8577 (JP); OGURA Izumi, Ashigarakami-gun, Kanagawa 258-8577 (JP)
(74) Representative: Hoffmann Eitle
(86) International application number: PCT/JP2019/029440
(87) International publication number: WO 2020/022483

(56) References cited:
- WO-A1-2006/126574
- WO-A1-2014/132933
- WO-A1-2014/132933
- WO-A1-2016/147975
- WO-A1-2017/154795
- WO-A1-2017/154795
- KABEYA TOMOKI ET AL: "Pharmacokinetic functions of human induced pluripotent stem cell-derived small intestinal epithelial cells", vol. 35, no. 4, 16 May 2020 (2020-05-16), JP, pages 374 - 382, XP055780291, ISSN: 1347-4367, Retrieved from the Internet <URL:https://www.sciencedirect.com/science/article/pii/S1347436720303621/pdfft?md5=9c27a7cd92acac102d0025f5e61ce72b&pid=1-s2.0-S1347436720303621-main.pdf> DOI: 10.1016/j.dmpk.2020.04.334
- TAKEDA J ET AL: "Forskolin activates adenylate cyclase activity and inhibits mitosis in in vitro in pig epidermis", THE JOURNAL OF INVESTIGATIVE DERMATOLOGY 81, 1 January 1983 (1983-01-01), pages 236 - 240, XP093109010, Retrieved from the Internet <URL:https://www.sciencedirect.com/science/article/pii/S0022202X15431744?via%3Dihub> [retrieved on 20231205]
- HOSONO M ET AL: "Cardiovascular and adenylate cyclase stimulant properties of NKH477, a novel water-soluble forskolin derivative", J CARDIOVASC PHARMACOL. 19(4), 1 April 1992 (1992-04-01), XP093109007, Retrieved from the Internet <URL:https://pubmed.ncbi.nlm.nih.gov/1380607/> [retrieved on 20231205]
- WIRTSHAFTER ET AL: "Rotation and immediate-early gene expression in rats treated with the atypical D1 dopamine agonist SKF 83822", PHARMACOLOGY BIOCHEMISTRY AND BEHAVIOR, ELSEVIER, US, vol. 86, no. 3, 27 March 2007 (2007-03-27), pages 505 - 510, XP022002229, ISSN: 0091-3057, DOI: 10.1016/J.PBB.2007.01.011
- CASSEL DAN ET AL: "Mechanism of adenylate cyclase activation by cholera toxin: Inhibition of GTP hydrolysis at the regulatory site (3':5'-cyclic AMP/guanyl nucleotide site/catecholamine-stimulated guanosinetriphosphatase/hormone", PNAS 74(8), 1 August 1977 (1977-08-01), pages 3307 - 3311, XP093109012, Retrieved from the Internet <URL:https://www.ncbi.nlm.nih.gov/pmc/articles/PMC431542/pdf/pnas00030-0209.pdf> [retrieved on 20231205]
- BOBECK ERIN N ET AL: "Contribution of Adenylyl Cyclase Modulation of Pre- and Postsynaptic GABA Neurotransmission to Morphine Antinociception and Tolerance", NEUROPSYCHOPHARMACOLOGY, vol. 39, no. 9, 9 April 2014 (2014-04-09), Cham, pages 2142 - 2152, XP093109013, ISSN: 0893-133X, Retrieved from the Internet <URL:https://www.ncbi.nlm.nih.gov/pmc/articles/PMC4104331/pdf/npp201462a.pdf> DOI: 10.1038/npp.2014.62

## Description

### Field of the Invention

The present invention relates to a method for producing an intestinal epithelial cell from a pluripotent stem cell and an intestinal epithelial cell produced from a pluripotent stem cell as defined in the appended claims.

### Description of the Related Art

Since many drug metabolizing enzymes and drug transporters are present in small intestine, the small intestine is very important as an organ involved in the first-pass effect of drugs, like the liver. Accordingly, evaluating the metabolism and the membrane permeability in the small intestine is important in research fields of the pharmaceutics and the food. At present, Caco-2 cells derived from human colon cancer are frequently used as a small intestine model system. However, the expression pattern of a drug transporter in Caco-2 cells is different from that in the human small intestine. In addition, it is difficult to accurately evaluate the pharmacokinetics in the small intestine, since Caco-2 cells hardly exhibit the expression and induction of a drug metabolizing enzyme. Accordingly, it is desirable to use primary small intestine epithelial cells in order to comprehensively evaluate the drug metabolism and membrane permeability in the small intestine, but it is difficult to obtain intestinal epithelial cells from the small intestine of animals, especially humans.

Human induced pluripotent stem (iPS) cells were established by Yamanaka et al. in 2007. The human iPS cells are cells having multiple differentiation potency and substantially infinite proliferation ability that are similar to those of human embryonic stem (ES) cells established by Thomson et al. in 1998. The human iPS cells have few ethical problems as compared with the human ES cells and are expected to be a stable cell source for the drug development. Accordingly, studies are underway to induce the differentiation of intestinal epithelial cells from pluripotent stem cells.

Patent Document 1 discloses inducing the differentiation of an induced pluripotent stem cell into an intestinal epithelial cell by a step that is a step of differentiating an induced pluripotent stem cell into an endodermal cell, a step of differentiating the endodermal cell obtained in the above step into an intestinal stem cell, and a step of differentiating the intestinal stem cell obtained in the above step into an intestinal epithelial cell, which includes culture in the presence of one or more compounds selected from the group consisting of a MEK1 inhibitor, a DNA methylation inhibitor, and a TGFβ receptor inhibitor, and EGF.

Patent Document 2 discloses inducing the differentiation of an induced pluripotent stem cell into an intestinal epithelial cell by a step that is a step (1) of differentiating an induced pluripotent stem cell into an endodermal cell, a step (2) of differentiating the endodermal cell obtained in the step (1) into an intestinal stem cell, and a step (3) of differentiating the intestinal stem cell obtained in the step (2) into an intestinal epithelial cell, which includes culture under the conditions of the presence of a MEK1 inhibitor, a DNA methylation inhibitor, a TGFβ receptor inhibitor, and EGF and the conditions under which cAMP is supplied to cells.

### Prior Art Documents

### Patent Documents

Patent Document 1: WO2014/132933A
Patent Document 2: WO2017/154795A

### SUMMARY OF THE INVENTION

For the induction of differentiation of a pluripotent stem cell into an intestinal epithelial cell, it is generally necessary to continuously carry out the culture medium exchange and the addition of reagents for several days to several tens of days. From the viewpoint of operational efficiency, it is desirable to perform the operation of the induction of differentiation using a large culture vessel or dish. On the other hand, in a case where a permeability test is carried out using intestinal epithelial cells, it is necessary to form an intestinal epithelial cell layer on a porous membrane mounted in a relatively small culture container called a cell culture insert.

In a case where a pluripotent stem cell is induced to differentiate into an intestinal epithelial cell in a large culture vessel or dish, and then the cell is replated on the porous membrane on the cell culture insert, there is concern that the barrier function required for the permeability test will be lost due to the possibility that the intestinal epithelial cell layer is not sufficiently formed. In a case where the differentiation of the pluripotent stem cell into an intestinal epithelial cell is induced on the porous membrane on the cell culture insert from the beginning, the intestinal epithelial cell layer is formed, but the culture operation is inefficient.

Accordingly, there has been a demand for a culture method capable of performing replating while maintaining the barrier function of the intestinal epithelial cell.

In addition, it is known that the conditions for the induction of differentiation into the intestinal epithelial cell are similar to those into the liver cell, and there is a concern that some cells differentiate into liver cells in the process of inducing pluripotent stem cells to intestinal epithelial cells. In a case where intestinal epithelial cells are contaminated with liver cells, the test results may affect by the contamination, and thus there has been a demand for a method of reducing the number of liver cells that contaminate the intestinal epithelial cells.

An object of the present invention is to provide a method for producing an intestinal epithelial cell in which the barrier function is maintained while the differentiation of a pluripotent stem cell into a liver cell is suppressed. Another object of the present invention is to provide an intestinal epithelial cell in which the barrier function is maintained while the differentiation into a liver cell is suppressed.

As a result of diligent studies, the inventors of the present invention have found that the problems described above can be solved by replating a cell under differentiation one or more times at the predetermined timing during the step of differentiating into an intestinal epithelial cell in a case of producing the intestinal epithelial cell by a step of differentiating a pluripotent stem cell into an intestinal stem cell and a step of differentiating the intestinal stem cell into an intestinal epithelial cell in the presence of one or more selected from the group consisting of a MEK1 inhibitor, a DNA methylation inhibitor, and a TGFβ receptor inhibitor, and EGF. The present invention has been completed based on these findings.

That is, according to the present invention, the following inventions are provided.
1. A method for producing an intestinal epithelial cell, comprising:
   a step 1-1 of differentiating a pluripotent stem cell into an endodermal cell,
      a step 1-2 of differentiating the endodermal cell into an intestinal stem cell;
      a step of treating the intestinal stem cell with ROCK inhibitor;
      a step of replating the treated intestinal stem cell; and
   a step 2 of differentiating the intestinal stem cell obtained in the step 1 into an intestinal epithelial cell in a presence of EGF, a cAMP activator and one or more selected from the group consisting of a MEK1 inhibitor, a DNA methylation inhibitor, and a TGFβ receptor inhibitor,
      wherein the period of step 1-1 is 2 days to 7 days, the period of step 1-2 is 3 days to 7 days, and the period of step 2 is 7 days to 40 days, and
   wherein during the step 2, a cell under differentiation is replated one or more times at the following timings while maintaining the barrier function;
      (a) a timing after 4th day after a start of the step 2 and 5 days or more before an end of the step 2,
   wherein the cAMP activator is Forskolin, indomethacin, NKH477 (colforsin daropate), pertussis toxin, cholera toxin, PACAP-27, PACAP-38, or SKF8382.
2. The method according to item 1,
   wherein during the step 2, the cell under differentiation is replated one or more times at the following timings;
   (a) a timing after 10th day after the start of the step 2 and before 5 days or more from an end of the step 2.
3. The method according to item 1 or 2,
   wherein the step 2 includes a step of differentiating the intestinal stem cell obtained in the step 1 into the intestinal epithelial cell in a presence of a MEK1 inhibitor, a DNA methylation inhibitor, a TGFβ receptor inhibitor, EGF, and a cAMP activator.
4. The method according to any one of items 1 to 3,
   wherein in the step 2, the cell is replated on a porous membrane.
5. The method according to any one of items 1 to 4,
   wherein the steps 1-1 and 1-2 are performed on a culture plate having a surface area of 30 cm² or more.
6. The method according to any one of items 1 to 5,
   wherein culture after the replating in the step 2 is performed on a culture plate having a surface area of 3 cm² or less per well.

According to an aspect of the present invention, it is possible to produce an intestinal epithelial cell in which the barrier function is maintained while the differentiation of a pluripotent stem cell into a liver cell is suppressed. In the intestinal epithelial cell produced according to an aspect of the present invention, the barrier function is maintained and the contamination of liver cells is suppressed.

### BRIEF DESCRIPTION OF THE DRAWINGS

Fig. 1 illustrates an outline of the differentiation culture methods of Comparative Examples 1 to 3 and Examples 1 to 4.
Fig. 2 shows the results of measuring the expression levels of small intestine markers over time. The vertical axis indicates relative expression levels of small intestine markers in a case where the expression level thereof in Adult Intestine is each set to 100, and the horizontal axis indicates the number of days after the start of differentiation of a pluripotent stem cell.

### DESCRIPTION OF THE PREFERRED EMBODIMENTS

Hereinafter, the embodiments of the present invention will be described in detail.

### [Explanation of terms]

MEK1: Mitogen-activated protein kinase kinase 1
TGFβ receptor: Transforming Growth Factor β receptor
EGF: Epidermal Growth Factor
LIF: Leukemia Inhibitory Factor
bFGF: basic fibroblast Growth Factor
SCF: Stem Cell Factor
FGF2: Fibroblast Growth Factor 2
GSK-3β: Glycogen Synthase Kinase 3β
cAMP: cyclic Adenosine Monophosphate
ROCK: Rho-associated coiled-coil forming kinase / Rho-binding kinase
BMP4: Bone Morphogenetic Protein 4
VEGF: Vascular Endothelial Growth Factor
FBS: Fetal Bovine Serum

### [Method for producing intestinal epithelial cell]

A method for producing an intestinal epithelial cell according to the embodiment of the present invention includes inducing the differentiation of a pluripotent stem cell into an intestinal epithelial cell lineage. According to the present invention, cells that exhibit the characteristics similar to intestinal epithelial cells which constitute the intestinal tissue of the living body, that is, intestinal epithelial cells are obtained.

The method for producing an intestinal epithelial cell according to the embodiment of the present invention includes a step 1 of differentiating a pluripotent stem cell into an intestinal stem cell; and a step 2 of differentiating the intestinal stem cell obtained in the step 1 into an intestinal epithelial cell in a presence of one or more selected from the group consisting of a MEK1 inhibitor, a DNA methylation inhibitor, and a TGFβ receptor inhibitor, and EGF as defined in the appended claims. In the method for producing an intestinal epithelial cell according to the embodiment of the present invention, during the step 2, the cell under differentiation is replated one or more times at the following timings;
(a) a timing after 4th day after a start of the step 2 and 5 days or more before an end of the step 2, as defined in the appended claims.

By replating one or more times at any of the timings described above, it is possible to replate the cells while maintaining the barrier function. As a result, it is possible to subdivide and replate the cells on the porous membrane on the cell culture insert after efficiently culturing in a large culture vessel or dish up to the middle of the culture, and thus the culture operation can be performed more efficiently.

Further, by replating one or more times at any of the timings described above, it is possible to significantly reduce the number of liver cells that contaminate the intestinal epithelial cell.

"Pluripotent stem cells" refer to cells having the ability (differentiation pluripotency) to differentiate into all cells that constitute a living body and the ability (self-renewal ability) to generate daughter cells having the same differentiation potential as that of the pluripotent stem cells through cell division. Differentiation pluripotency can be evaluated by transplanting the cells to be evaluated into nude mice and testing for the presence or absence of formation of teratoma including cells of each of the three germ layers (ectoderm, mesoderm, and endoderm).

As the pluripotent stem cells, embryonic stem cells (ES cells), embryonic germ cells (EG cells), induced pluripotent stem cells (iPS cells), and the like can be mentioned, but the pluripotent stem cells are not limited thereto as long as they have differentiation pluripotency and self-renewal ability. ES cells or iPS cells are preferably used. iPS cells are more preferably used. The pluripotent stem cells are preferably mammalian (for example, primates such as a human and a chimpanzee, rodents such as a mouse and a rat) cells and particularly preferably human cells. Accordingly, in the most preferred embodiment of the present invention, human iPS cells are used as pluripotent stem cells.

Non-human ES cells are established by culturing an early embryo before implantation, an inner cell mass constituting the early embryo, a single blastomere, or the like (Manipulating the Mouse Embryo A Laboratory Manual, Second Edition, Cold Spring Harbor Laboratory Press (1994); Thomson, J. A. et. al., Science, 282, 1145-1147 (1998)). As the non-human early embryo, an early embryo produced by nuclear transfer of a somatic cell nucleus may be used (Wilmut et al. (Nature, 385, 810 (1997)), Cibelli et al. (Science, 280, 1256 (1998)), Iriya et al. ((Protein Nucleic Acid Enzyme, 44, 892 (1999)), Baguisi et al. (Nature Biotechnology, 17, 456 (1999)), Wakayama et al. (Nature, 394, 369 (1998)); Nature Genetics, 22, 127 (1999); Proc. Natl. Acad. Sci. USA, 96, 14984 (1999), RideoutIII et al. (Nature Genetics, 24, 109 (2000), Tachibana et al. (Human Embryonic Stem Cells Derived by Somatic Cell Nuclear Transfer, Cell (2013) in press)). As early embryo, a parthenogenetic embryo may be used (Kim et al. (Science, 315, 482-486 (2007)), Nakajima et al. (Stem Cells, 25, 983-985 (2007)), Kim et al. (Cell Stem Cell, 1, 346-352 (2007)), Revazova et al. (Cloning Stem Cells, 9, 432-449 (2007)), Revazova et al. (Cloning Stem Cells, 10, 11-24 (2008)). In addition to the above-mentioned papers, the production of ES cells is described in Strelchenko N., et al. Reprod Biomed Online. 9: 623-629, 2004; KlimanskayaI., et al. Nature 444: 481-485, 2006; Chung Y., et al. Cell Stem Cell 2: 113-117, 2008; Zhang X., et al Stem Cells 24:2669-2676, 2006; Wassarman, P. M. et al. Methods in Enzymology, Vol. 365, 2003, or the like. In addition, non-human fused ES cells obtained by cell fusion of ES cells with somatic cells are also included in the embryonic stem cells used in the method of the present invention.

Some ES cells are available from conservation institutions or are commercially available. For example, human ES cells are available from the Institute for Frontier Medical Sciences, Kyoto University (for reference only KhES-1, KhES-2, and KhES-3), WiCell Research Institute, ESIBIO, and the like.

The EG cells can be established by culturing primordial germ cells in the presence of LIF, bFGF, and SCF (Matsui et al., Cell, 70, 841-847 (1992), Shamblott et al., Proc. Natl. Acad. Sci. USA, 95 (23), 13726-13731 (1998), Turnpenny et al., Stem Cells, 21 (5), 598-609, (2003)).

"Induced pluripotent stem cells (iPS cells)" are cells that have pluripotency (multiple differentiation potency) and proliferation ability and that are produced by reprogramming somatic cells by introducing reprogramming factors or the like. The induced pluripotent stem cells exhibit properties similar to the ES cells. The somatic cells used for producing iPS cells are not particularly limited and may be differentiated somatic cells or undifferentiated stem cells. In addition, the origin of the somatic cells is not particularly limited but preferably somatic cells of mammals (for example, primates such as a human and a chimpanzee, rodents such as a mouse and a rat) and particularly preferably human somatic cells. The iPS cells can be produced by various methods reported so far. In addition, it is naturally expected that an iPS cell production method to be developed in the future will be applied.

The most basic method for producing iPS cells is a method in which four transcription factors, Oct3/4, Sox2, Klf4, and c-Myc are introduced into cells using a virus (Takahashi K, Yamanaka S: Cell 126 (4), 663-676, 2006; Takahashi, K, et al: Cell 131 (5), 861-72, 2007). It has been reported that human iPS cells have been established by introducing four factors, Oct4, Sox2, Lin28, and Nanog (Yu J, et al: Science 318 (5858), 1917-1920, 2007). It has also been reported that iPS cells have been established by introducing three factors excluding c-Myc (Nakagawa M, et al: Nat. Biotechnol. 26 (1), 101-106, 2008), two factors of Oct3/4 and Klf4 (Kim J B, et al: Nature 454 (7204), 646-650, 2008), or Oct3/4 alone (Kim J B, et al: Cell 136 (3), 411-419, 2009). In addition, a method of introducing a protein, which is an expression product of a gene, into cells (Zhou H, Wu S, Joo J Y, et al: Cell Stem Cell 4, 381-384, 2009; Kim D, Kim C H, Moon J I, et al: Cell Stem Cell 4, 472-476, 2009) has also been reported. On the other hand, it has also been reported that, by using BIX-01294 which is an inhibitor of histone methyltransferase G9a, valproic acid (VPA) which is a histone deacetylase inhibitor, or Bay K8644, the production efficiency has been improved and the factors to be introduced have been reduced (Huangfu D, et al: Nat. Biotechnol. 26 (7), 795-797, 2008; Huangfu D, et al: Nat. Biotechnol. 26 (11), 1269-1275, 2008; Silva J, et al: PLoS. Biol. 6 (10), e253, 2008). Studies on gene transfer methods have also been performed, and technologies for gene transfer have been developed using, in addition to a retrovirus, a lentivirus (Yu J, et al: Science 318 (5858), 1917-1920, 2007), an adenovirus (Stadtfeld M, et al: Science 322 (5903), 945-949, 2008), a plasmid (Okita K, et al: Science 322 (5903), 949-953, 2008), a transposon vector (Woltjen K, Michael I P, Mohseni P, et al: Nature 458, 766-770, 2009; Kaji K, Norrby K, Paca A, et al: Nature 458, 771-775, 2009; Yusa K, Rad R, Takeda J, et al: Nat Methods 6, 363-369, 2009), or an episomal vector (Yu J, Hu K, Smuga-Otto K, Tian S, et al: Science 324, 797-801, 2009).

Cells transformed to iPS cells, that is, cells that have undergone initialization (reprogramming) can be selected using, as an index, the expression of pluripotent stem cell markers (undifferentiated markers) such as Fbxo15, Nanog, Oct4, Fgf-4, Esg-1, and Cript, or the like. The selected cells are recovered as iPS cells.

The iPS cells can be available from, for example, National University Corporation Kyoto University, or Independent Administrative Institution RIKEN BioResource Center.

In the present specification, the "induction of differentiation" refers to acting to differentiate along a specific cell lineage. In the present invention, pluripotent stem cells are induced to differentiate into intestinal epithelial cells. The method for producing an intestinal epithelial cell according to the embodiment of the present invention roughly includes induction steps of two stages, that is, a step (step 1) of differentiating pluripotent stem cells into intestinal stem cells and a step (step 2) of differentiating the obtained intestinal stem cells into intestinal epithelial cells. Hereinafter, the details of each step will be described.

### <Step 1: Differentiation into intestinal stem cell>

In step 1, pluripotent stem cells are cultured and differentiated into intestinal stem cells. In other words, the pluripotent stem cells are cultured under the conditions that induce differentiation into intestinal stem cells. The culture conditions are not particularly limited as long as the pluripotent stem cells differentiate into intestinal stem cells. Typically, the induction of differentiation in two stages described below, that is, the differentiation of the pluripotent stem cells into endodermal cells (step 1-1) and the differentiation of the endodermal cells into intestinal stem cells (step 1-2) are performed such that the pluripotent stem cells differentiate into the intestinal stem cells via the endodermal cells.

### Step 1-1: Differentiation into endodermal cell

In this step, the pluripotent stem cells are cultured and differentiated into endodermal cells. In other words, the pluripotent stem cells are cultured under the conditions that induce differentiation into endoderm. The culture conditions are not particularly limited as long as the pluripotent stem cells differentiate into endodermal cells. For example, the pluripotent stem cells are cultured in a culture medium to which activin A is added, according to a conventional method. In this case, the concentration of activin A in the culture medium is set to, for example, 10 ng/mL to 200 ng/mL and preferably 20 ng/mL to 150 ng/mL. It is preferable to add serum or a serum substitute (KnockOut^{™} Serum Replacement (KSR) or the like) to the culture medium from the viewpoints of cell growth rate, maintenance, and the like. The serum is not limited to fetal bovine serum, and human serum, sheep serum, or the like can be also used. The addition amount of serum or a serum substitute is, for example, 0.1% (v/v) to 10% (v/v).

An inhibitor of the Wnt/β-catenin signaling pathway (for example, hexachlorophene, quercetin, or Wnt3a which is a Wnt ligand) may be added to the culture medium to promote differentiation into endodermal cells.

This step can be also performed by the method described in WO2014/165663A or a method based thereon.

The period (culture period) of the step 1-1 is, 2 days to 7 days.

### Step 1-2: Differentiation into intestinal stem cells

In this step, the endodermal cells obtained in step 1-1 are cultured and differentiated into intestinal stem cells. In other words, the endodermal cells are cultured under the conditions that induce differentiation into intestinal stem cells. The culture conditions are not particularly limited as long as the endodermal cells differentiate into intestinal stem cells. The culture is preferably performed in the presence of FGF2 or the presence of a GSK-3β inhibitor. Human FGF2 (for example, a human recombinant FGF2) is preferably used as FGF2.

Typically, the cell population or a part thereof obtained through the step 1-1 is used in the step 1-2 without selection. Alternatively, the step 1-2 may be performed after selecting endodermal cells from the cell population obtained through step 1-1. The selection of endodermal cells may be performed, for example, with a flow cytometer (cell sorter) using a cell surface marker as an index.

"In the presence of FGF2" is synonymous with under the condition in which FGF2 is added to a culture medium. Accordingly, in order to perform culture in the presence of FGF2, a culture medium to which FGF2 is added may be used. An example of the concentration of FGF2 to be added is 100 ng/mL to 500 ng/mL.

Similarly, "in the presence of a GSK-3β inhibitor" is synonymous with under the conditions in which a GSK-3β inhibitor has been added to a culture medium. Accordingly, in order to perform culture in the presence of GSK-3β inhibitor, a culture medium to which GSK-3β inhibitor has been added may be used. Examples of the GSK-3β inhibitor include CHIR99021, SB216763, CHIR98014, TWS119, Tideglusib, SB415286, BIO, AZD2858, AZD1080, AR-A014418, TDZD-8, LY2090314, IM-12, Indirubin, Bikinin, and 1-Azakenpaullone. An example of the concentration of the GSK-3β inhibitor to be added (in the case of CHIR99021) is 1 µmol/L to 100 µmol/L and preferably 3 µmol/L to 30 µmol/L.

The period (culture period) of the step 1-2 is 3 days to 7 days. In a case where the culture period is too short, an expected effect (increase in differentiation efficiency or promotion of acquisition of functions as intestinal stem cells) cannot be sufficiently obtained. On the other hand, in a case where the culture period is too long, the differentiation efficiency will be reduced.

The differentiation into intestinal stem cells can be determined or evaluated using, for example, the expression of an intestinal stem cell marker as an index. Examples of the intestinal stem cell marker include G protein-coupled receptor 5 (LGR5) containing leucine-rich repeats and Ephrin B2 receptor (EphB2).

### <Step 2: Differentiation into intestinal epithelial cell>

In the step 2, the intestinal stem cells obtained in the step 1 are differentiated into intestinal epithelial cells by using one or more selected from the group consisting of a MEK1 inhibitor, a DNA methylation inhibitor, and a TGFβ receptor inhibitor, and EGF in combination. As "one or more selected from the group consisting of a MEK1 inhibitor, a DNA methylation inhibitor and a TGFβ receptor inhibitor", one inhibitor among a MEK1 inhibitor, a DNA methylation inhibitor, and a TGFβ receptor inhibitor (that is, any one of a MEK1 inhibitor, a DNA methylation inhibitor, or a TGFβ receptor inhibitor) may be adopted, two inhibitors among a MEK1 inhibitor, a DNA methylation inhibitor, and a TGFβ receptor inhibitor (that is, a combination of a MEK1 inhibitor and a DNA methylation inhibitor, a combination of a MEK1 inhibitor and a TGFβ receptor inhibitor, or a combination of a DNA methylation inhibitor and a TGFβ receptor inhibitor) may be adopted, or all inhibitors of a MEK1 inhibitor, a DNA methylation inhibitor, and a TGFβ receptor inhibitor may be adopted.

The step 2 particularly preferably includes a step of differentiating the intestinal stem cells obtained in the step 1 into the intestinal epithelial cell in the presence of a MEK1 inhibitor, a DNA methylation inhibitor, a TGFβ receptor inhibitor, EGF, and a cAMP activator.

Typically, the cell population or a part thereof obtained through the step 1 is used in the step 2 without selection. Alternatively, the step 2 may be performed after selecting intestinal stem cells from the cell population obtained through step 1. The selection of intestinal stem cells may be performed, for example, with a flow cytometer (cell sorter) using a cell surface marker as an index.

The step 2 is constituted of one or two or more cultures (details will be described later). In each culture constituting the step 2, culture media such as a culture medium to which EGF and a cAMP activator have been added as essential components, a culture medium to which EGF, a cAMP activator, a DNA methylation inhibitor, a MEK1 inhibitor, and a TGFβ receptor inhibitor have been added as essential components, a culture medium to which EGF has been added as an essential component, and a culture medium to which EGF and a ROCK inhibitor have been added as essential components are used.

As the MEK1 inhibitor, PD98059, PD184352, PD184161, PD0325901, U0126, MEK inhibitor I, MEK inhibitor II, MEK1/2 inhibitor II, and SL327 can be mentioned.

As the DNA methylation inhibitor, 5-aza-2'-deoxycytidine (5-aza-2'dc), 5-azacytidine, RG108, and zebularine can be mentioned.

Regarding the TGFβ receptor inhibitor, considering that A-83-01 used in Examples described later exhibits an inhibitory activity on TGF-β receptors ALK4, ALK5, and ALK7, it is preferable to use an inhibitor that exhibits an inhibitory activity on one or more of TGF-β receptors, ALK4, ALK5, and ALK7. For example, A8301, SB431542, SB-505124, SB525334, D4476, ALK5 inhibitor, LY2157299, LY364947, GW788388, and RepSox satisfy the above conditions.

As the cAMP activator, Forskolin, indomethacin, NKH477 (colforsin daropate), a cell-derived toxin protein (pertussis toxin, cholera toxin), PACAP-27, PACAP-38, or SKF83822 are used. Forskolin exhibits an adenylate cyclase activating activity and promotes the intracellular cAMP synthesis.

An example of the concentration of the MEK1 inhibitor to be added (in the case of PD98059) is 4 µmol/L to 100 µmol/L and preferably 10 µmol/L to 40 µmol/L. An example of the concentration of the DNA methylation inhibitor (in the case of 5-aza-2'-deoxycytidine) to be added is, 1 µmol/L to 25 µmol/L and preferably 2.5 µmol/L to 10 µmol/L, and an example of the concentration of the TGFβ receptor inhibitor (in the case of A8301) to be added is, for example, 0.1 µmol/L to 2.5 µmol/L and preferably 0.2 µmol/L to 1 µmol/L. An example of the concentration of EGF to be added is 5 ng/mL to 100 ng/mL and preferably 10 ng/mL to 50 ng/mL. An example of the concentration of the CAMP activator to be added (in the case of Forskolin) is 1 µmol/L to 200 µmol/L and preferably 5 µmol/L to 100 µmol/L. However, the concentration of compounds in a case of using compounds different from the exemplified compounds, that is, PD98059, 5-aza-2'-deoxycytidine, A-83-01, and Forskolin, can be set according to the above-described concentration range by those skilled in the art in consideration of the difference in the properties (especially the difference in activity) between the compound to be used and the exemplified compounds (PD98059, 5-aza-2'-deoxycytidine, A-83-01, and Forskolin). Whether the set concentration range is suitable or not can be confirmed by a preliminary experiment according to Examples described later.

The period (culture period) of the step 2 is, 7 days to 40 days and preferably 10 days to 30 days. In a case where the culture period is too short, an expected effect (increase in differentiation efficiency or promotion of acquisition of functions as intestinal epithelial cells) cannot be sufficiently obtained. On the other hand, in a case where the culture period is too long, the differentiation efficiency will be reduced.

The differentiation into intestinal epithelial cells can be determined or evaluated using, for example, the expression of an intestinal epithelial cell marker, the incorporation of a peptide, or the induction of the expression of a drug metabolizing enzyme via a vitamin D receptor as an index. Examples of the intestinal epithelial cell marker include Villin 1, CDX2, Intestine-specific homeobox (ISX), ATP-binding cassette transporter B1 / Multidrug resistance protein 1 (ABCB1/MDR1), ATP-binding cassette transporter G2 / Breast cancer resistance protein (ABCG2/BCRP), cytochrome P4503A4 (CYP3A4), Fatty acid binding protein 2 (FABP2), Pregnane X receptor (PXR), Solute carrier (SLC) family member 5A1 / Sodium-coupled glucose transporter 1 (SLC5A1/SGLT1), Solute carrier (SLC) family member 15A1 / Peptide transporter 1 (SLC15A1/PEPT1), Solute carrier (SLC) organic anion transporter 2B1 (SLCO2B1/OATP2B1), Sucrase-isomaltase, Uridine diphosphate glucuronosyltransferase 1A1 (UGT1A1), Uridine diphosphate glucuronosyltransferase 1A4 (UGT1A4), and Carboxylesterase 2A1 (CES2A1). Among these, Villin 1, CDX2, Intestine-specific homeobox (ISX) are particularly effective markers.

To obtain a cell population consisting only of target cells (intestinal epithelial cells) or a cell population including the target cells in a high proportion (high purity), the cell population after culture may be selected and sorted using a cell surface marker characteristic of the target cells as an index.

As the step 2, any one of the following culture steps A to D is preferably performed.

### <Culture step A>

In culture step A, a culturing (a-1) in the presence of EGF and a cAMP activator and a culturing (a-2) in the presence of a MEK1 inhibitor, a DNA methylation inhibitor, a TGFβ receptor inhibitor, and EGF, which is performed after the culturing (a-1). In a case of performing the two-stage culture in this manner, the effects of the promotion of differentiation into intestinal epithelial cells, maturation, and the acquisition of functions can be expected. The period of the culturing (a-1) is, for example, 2 days to 10 days and preferably 4 days to 8 days, and the period of the culturing (a-2) is, for example, 9 days to 29 days and preferably 7 days to 27 days. For items not particularly described (compounds usable for each culture, the concentration of each compound to be added, and the like), the corresponding description described above is cited.

### <Culture step B>

In culture step B, a culturing (b-1) in the presence of EGF and a culturing (b-2) in the presence of a MEK1 inhibitor, a DNA methylation inhibitor, a TGFβ receptor inhibitor, EGF, and a cAMP activator, which is performed after the culturing (b-1) are performed. In a case of performing the two-stage culture in this manner, the effects of the promotion of differentiation into intestinal epithelial cells, maturation, and the acquisition of functions can be expected. The period of the culturing (b-1) is, for example, 2 days to 10 days and preferably 4 days to 8 days, and the period of the culturing (b-2) is, for example, 9 days to 19 days and preferably 7 days to 17 days. For items not particularly described (compounds usable for each culture, the concentration of each compound to be added, and the like), the corresponding description described above is cited.

After the culturing (b-2), culture (culturing (b-3)) in the presence of a MEK1 inhibitor, a DNA methylation inhibitor, a TGFβ receptor inhibitor, and EGF may be performed. The period of this culture is, for example, 1 day to 10 days. In a case of performing this culture, the effects of the promotion of differentiation into intestinal epithelial cells, maturation, and the acquisition of functions can be expected.

### <Culture step C>

In culture step C, a culturing (c-1) in the presence of EGF and a cAMP activator and a culturing (c-2) in the presence of a MEK1 inhibitor, a DNA methylation inhibitor, a TGFβ receptor inhibitor, EGF, and a cAMP activator, which is performed after the culturing (c-1) are performed. In a case of performing the two-stage culture in this manner, the effects of the promotion of differentiation into intestinal epithelial cells, maturation, and the acquisition of functions can be expected. The period of the culturing (c-1) is, for example, 2 days to 10 days and preferably 4 days to 8 days, and the period of the culturing (c-2) is, for example, 9 days to 19 days and preferably 7 days to 17 days. For items not particularly described (compounds usable for each culture, the concentration of each compound to be added, and the like), the corresponding description described above is cited.

After the culturing (c-2), culture (culturing (c-3)) in the presence of a MEK1 inhibitor, a DNA methylation inhibitor, a TGFβ receptor inhibitor, and EGF may be performed. The period of this culture is, for example, 1 day to 10 days. In a case of performing this culture, the effects of the promotion of differentiation into intestinal epithelial cells, maturation, and the acquisition of functions can be expected.

### <Culture step D>

In culture step D, a culturing (d-1) in the presence of a MEK1 inhibitor, a DNA methylation inhibitor, a TGFβ receptor inhibitor, EGF, and a cAMP activator. This culture step is particularly advantageous in that the culture operation is simple, the differentiation into intestinal epithelial cells is more effective, and a stable effect can be expected since chemical compounds are used. The period of the culturing (d-1) is, for example, 15 days to 25 days and preferably 17 days to 23 days. For items not particularly described (compounds usable for each culture, the concentration of each compound to be added, and the like), the corresponding description described above is cited.

After the culturing (d-1), culture (culturing (d-2)) in the presence of a MEK1 inhibitor, a DNA methylation inhibitor, a TGFβ receptor inhibitor, and EGF may be performed. The period of this culture is, for example, 1 day to 10 days. In a case of performing this culture, the effects of the promotion of differentiation into intestinal epithelial cells, maturation, and the acquisition of functions can be expected.

In each of the steps (step 1, step 1-1, step 1-2, step 2, step a-1, step a-2, step b-1, step b-2, step b-3, step c-1, step c-2, step c-3, step d-1, and step d-2) which can constitute the present invention as defined in the appended claims, subculture (replating) may be carried out in the middle of each step.

For example, in a case where cells become confluent or subconfluent, a part of the cells are collected and transferred to another culture vessel, and the culture is continued. It is preferable to set a cell density low in order to promote differentiation. For example, cells may be plated at a cell density of about 1 × 10⁴ cells/cm² to 1 × 10⁶ cells/cm².

At the time of cell recovery associated with culture medium exchange or subculture, it is better to treat the cells in advance with a ROCK inhibitor such as Y-27632 in order to suppress cell death. Accordingly, in a case of replating the cell under differentiation one or more times, a culture medium for replating during the step 2 is preferably a culture medium containing a ROCK inhibitor.

In the present invention, during the step 2, the cell under differentiation is replated one or more times at any one of the following timings:
(a) a timing after 4th day after a start of the step 2 and 5 days or more before an end of the step 2 (preferably a timing after 10th day after a start of the step 2 and 5 days or more before an end of the step 2, and more preferably a timing after 11th day after a start of the step 2 and 5 days or more before an end of the step 2);

In the step 2, the cell is preferably replated on a porous membrane. By replating the cells on the porous membrane, the drug metabolism and membrane permeability can be directly evaluated with the obtained intestine epithelial cells. The porous membrane refers to a membrane having penetrating pores. As the porous membrane, for example, a polycarbonate membrane, a polyethylene terephthalate (PET) membrane, or the like, having a large number of pores having a pore diameter of about 0.4 to 1.0 µm, can be used.

In the present invention, the step 1 is preferably performed on a culture plate having a surface area of 30 cm² or more. That is, it is desirable for the operation of the induction of differentiation in the step 1 to be performed by using a large culture vessel or dish, which can improve the efficiency of the operation. In addition, in a case where a permeability test is carried out using intestinal epithelial cells, it is necessary to form an intestinal epithelial cell layer on a porous membrane mounted in a relatively small culture container called a cell culture insert. For this reason, it is preferable to carry out the culture after the replating in the step 2 on a culture plate (more preferably on a porous membrane) having a surface area of 3 cm² or less per well.

Other culture conditions (such as the culture temperature) in the individual steps constituting the present invention may be conditions generally employed in culturing animal cells. That is, culture may be performed, for example, at 37°C in an environment of 5% CO₂. In addition, as a basic culture medium, Iskov modified Dulbecco's medium (IMDM) (GIBCO-BRL or the like), Ham F12 medium (Ham F12) (SIGMA, Gibco-BRL, or the like), Dulbecco's modified Eagle's medium (D-MEM) (Nacalai Tesque Inc., Sigma-Aldrich Co. LLC, Gibco-BRL or the like), Glasgow basic culture medium (Gibco-BRL or the like), RPMI1640 medium, or the like can be used. Two or more basic culture media may be used in combination. In the step 1-2, the step 2, and the culture step A, the culture step B, the culture step C, and the culture step D which constitute the step 2, a basic culture medium suitable for culturing epithelial cells (for example, a mixed culture medium of D-MEM and Ham F12 medium, and D-MEM) can be preferably used. Examples of components that can be added to the culture medium include bovine serum albumin (BSA), an antibiotic, 2-mercaptoethanol, polyvinyl alcohol (PVA), non-essential amino acids (NEAA), insulin, transferrin, and selenium. Typically, cells are two-dimensionally cultured using a culture dish or the like. The method according to the embodiment of the present invention makes it possible to obtain an intestinal epithelial cell from a pluripotent stem cell by two-dimensional culture. In addition, three-dimensional culture may be performed using a gel-like culture substrate or a three-dimensional culture plate.

### [Intestinal epithelial cell]

According to the present invention, an intestinal epithelial cell obtained by the above-described method for producing an intestinal epithelial cell according to the embodiment of the present invention is provided. The intestinal epithelial cell produced according to the embodiment of the present invention is preferably a cell in which the expression level of albumin, which is a liver marker, is equal to or less than the expression level of albumin in Caco-2 cell.

The present disclosure further relates to the use of the intestinal epithelial cell obtained by the method according to the embodiment of the present invention. Various assays are provided as the first use. The intestinal epithelial cell provided according to the embodiment of the present invention can be used for a model system of the intestinal tract, particularly the small intestine, and are useful for evaluating pharmacokinetics (absorption, metabolism, and the like) and toxicity in the intestinal tract, particularly the small intestine. In other words, the intestinal epithelial cell provided according to the embodiment of the present invention can be used for evaluating pharmacokinetics and toxicity of compounds.

Specifically, the intestinal epithelial cell provided according to the embodiment of the present invention can be used to test the absorbability or membrane permeability, drug interaction, induction of a drug metabolizing enzyme, induction of a drug transporter, toxicity, or the like with respect to the test substance. That is, the present invention provides a method (first aspect) for evaluating absorbability or membrane permeability, drug interaction, induction of a drug metabolizing enzyme, induction of a drug transporter, toxicity, or the like with respect to the test substance, as one of the uses of the intestinal epithelial cell. This method performs a step (i) of preparing a cell layer formed of the intestinal epithelial cells obtained by the production method according to the embodiment of the present invention, a step (ii) of bringing a test substance into contact with the cell layer; and a step (iii) of quantifying the test substance that has permeated the cell layer and evaluating absorbability or membrane permeability, drug interaction, induction of a drug metabolizing enzyme, induction of a drug transporter, or toxicity of the test substance. In addition, the absorbability of the test substance can also be evaluated by the method described below (second aspect).

In the step (i), intestinal epithelial cells are typically cultured on a semipermeable membrane (porous membrane) to form a cell layer. Specifically, for example, by using a culture vessel equipped with a cell culture insert (for example, Transwell (registered trademark) provided by Corning Incorporated), cells are plated and cultured in the cell culture insert, and then a cell layer constituted of the intestinal epithelial cells are obtained.

The cell culture insert is a culture vessel having a permeable membrane used for culturing a cell, an organ, or a tissue, and is mainly used in combination with a well plate. By culturing a cell, an organ, or a tissue in the cell culture insert, the components contained in the culture medium can be spread to the upper and lower surfaces of the organ and tissue, and the culture can be performed under conditions similar to those in the living body.

The "contact" in the step (ii) is typically performed by adding a test substance to a culture medium. The timing for adding the test substance is not particularly limited. Accordingly, the test substance may be added at a certain timing after starting culture in a culture medium containing no test substance, or the culture may be started in a culture medium containing the test substance in advance.

As the test substance, an organic compound or an inorganic compound having various molecular sizes can be used. Examples of the organic compound include a nucleic acid, a peptide, a protein, a lipid (a simple lipid, a complex lipid (a phosphoglyceride, a sphingolipid, a glycosylglyceride, a cerebroside, or the like), a prostaglandin, an isoprenoid, a terpene, a steroid, a polyphenol, catechin, and a vitamin (B1, B2, B3, B5, B6, B7, B9, B12, C, A, D, E, or the like). Existing components or candidate components such as a pharmaceutical, a nutritional food, a food additive, a pesticide, and perfumery (a cosmetic) are also one of the suitable test substances. A plant extract, a cell extract, a culture supernatant or the like may be used as the test substance. By adding two or more test substances at the same time, the interaction, the synergism, or the like between the test substances may be examined. The test substance may be of natural origin or synthetic. In the latter case, an efficient assay system can be constructed using, for example, a combinatorial synthesis technique.

The period for bringing the test substance into contact can be appropriately set. The contact period is, for example, 10 minutes to 3 days and preferably 1 hour to 1 day. The contact may be performed a plurality of times.

In the step (iii), the test substance that has permeated the cell layer is quantified. For example, in a case where a culture vessel equipped with a cell culture insert such as Transwell (registered trademark) is used, a test substance that has permeated the cell culture insert, that is, the test substance that has migrated into the upper or lower vessel through the cell layer is quantified depending on the test substance by a measuring method such as mass spectrometry, liquid chromatography, an immunological method (for example, a fluorescent immunoassay method (fluoroimmunoassay (FIA) method) and an enzymatic immunoassay method (enzyme immunoassay (EIA) method), or the like. Based on the quantification results (the amount of the test substance permeated the cell layer) and the amount of the test substance used (typically, the amount added to the culture medium), the absorbability or membrane permeability, drug interaction, induction of a drug metabolizing enzyme, induction of a drug transporter, or toxicity are determined and evaluated with respect to the test substance.

The present disclosure also provides, as another aspect (second aspect), a method for evaluating metabolism or absorption of a test substance. In this method, a step (I) of bringing a test substance into contact with the intestinal epithelial cells obtained by the differentiation induction method according to the embodiment of the present invention and a step (II) of measuring and evaluating metabolism or absorption, drug interaction, induction of a drug metabolizing enzyme, induction of a drug transporter, or toxicity of the test substance are performed.

The step (I), that is, bringing the test substance into contact with intestinal epithelial cells can be performed in the same manner as in the step (ii). However, it is not essential to form a cell layer in advance.

After the step (I), the metabolism or absorption, drug interaction, induction of a drug metabolizing enzyme, induction of a drug transporter, or toxicity is measured and evaluated with respect to the test substance (step (II)). Metabolism or the like may be measured and evaluated, substantially without time interval, immediately after the step (I), that is, after bringing the test substance into contact with cells, or the metabolism or the like may be measured and evaluated after a certain time (for example, 10 minutes to 5 hours) has passed. The measurement of metabolism can be performed, for example, by detecting a metabolite. In this case, the expected metabolite is usually qualitatively or quantitatively measured using the culture solution after the step (I) as a sample. A suitable measurement method may be selected depending on the metabolite, and for example, mass spectrometry, liquid chromatography, and an immunological method (for example, a fluorescent immunoassay method (FIA method) and an enzymatic immunoassay method (EIA method)), or the like can be employed.

Typically, in a case where a metabolite of a test substance is detected, it is determined or evaluated that "the test substance has been metabolized". In addition, the metabolism quantity of the test substance can be evaluated depending on the amount of the metabolite. The metabolism efficiency of the test substance may be calculated based on the detection result of the metabolite and the used amount of the test substance (typically, the amount added to the culture medium).

The metabolism of a test substance can be measured using, as an index, the expression of drug metabolizing enzymes (cytochrome P450 (particularly CYP3A4), uridine diphosphate glucuronosyltransferase (particularly UGT1A8 or UGT1A10), and sulfotransferase (particularly SULT1A3 or the like)) in intestinal epithelial cells. The expression of drug metabolizing enzymes can be evaluated at the mRNA level or the protein level. For example, in a case where an increase in the mRNA level of a drug metabolizing enzyme is recognized, it can be determined that "the test substance has been metabolized". Similarly, in a case where an increase in the activity of a drug metabolizing enzyme is recognized, it can be determined that "the test substance has been metabolized". Similarly to the case where the metabolite is used as an index for determination, quantitative determination or evaluation may be performed based on the expression level of the drug metabolizing enzyme.

In order to evaluate the absorption of a test substance, for example, the remaining amount of the test substance in the culture solution is measured. Usually, the test substance is quantified using the culture solution after the step (I) as a sample. A suitable measuring method may be selected depending on the test substance. For example, mass spectrometry, liquid chromatography, and an immunological method (for example, a fluorescent immunoassay method (FIA method) and an enzymatic immunoassay method (EIA method), or the like can be employed. Typically, in a case where a decrease in the content of test substance in the culture solution is recognized, it is determined or evaluated that "the test substance has been absorbed". In addition, the absorption amount or the absorption efficiency of the test substance can be determined or evaluated depending on the degree of the decrease. The absorption can also be evaluated by measuring the amount of the test substance incorporated into the cells.

The measurement or evaluation of the metabolism and the measurement or evaluation of the absorption may be performed simultaneously or in parallel.

As a second use of the intestinal epithelial cell prepared by the differentiation induction method according to the embodiment of the present invention, a cell preparation containing intestinal epithelial cells is provided. The cell preparation can be applied to the treatment of various intestinal diseases. In particular, use as a material for regeneration/reconstruction of a damaged intestinal epithelial tissue (including dysfunction) is considered. That is, the contribution to regenerative medicine can be expected. The cell preparation can be prepared by, for example, suspending the intestinal epithelial cells obtained by the method according to the embodiment of the present invention in a physiological saline solution or a buffer (for example, a phosphate buffer) or producing a three-dimensional tissue (organoid or spheroid) using the intestinal epithelial cells. In order to be able to administer a therapeutically effective amount of cells, a single dose may contain, for example, 1 × 10⁵ to 1 × 10¹⁰ cells. The cell content can be suitably adjusted in consideration of the purpose of use, the target disease, the gender, age, weight, and state of the affected part of the target (recipient) to be administered, cell state, and the like.

Dimethyl sulfoxide (DMSO) and serum albumin for the protection of cells, an antibiotic for the prevention of bacterial contamination, and various components (a vitamin, a cytokine, a growth factor, a steroid, and the like) for the activation, proliferation, induction of differentiation, or the like of cells may be contained in the cell preparation. In addition, other pharmaceutically acceptable components (for example, a carrier, an excipient, a disintegrant, a buffer, an emulsifier, a suspending agent, a soothing agent, a stabilizer, a preservative, an antiseptic agent, physiological saline, and the like) may be contained in the cell preparation.

The present invention will be more specifically described with reference to Examples, but the present invention is not limited to the scope of Examples.

### Examples

### <Culture of human iPS cell>

Human iPS cells (FF-1) were plated on a dish coated with Matrigel (registered trademark) and cultured at 37°C in a CO₂ incubator under the condition of 5% O₂. Passage of human iPS cells (FF-1) was performed at a split ratio of 1:3 to 1:10 after culturing for 3 to 5 days. The culture medium was not changed for 48 hours after passage and thereafter was changed daily. Human iPS cells (FF-1) were obtained from FUJIFILM Cellular Dynamics, Inc.

### <Induction of human iPS cell differentiation into intestinal epithelial cell>

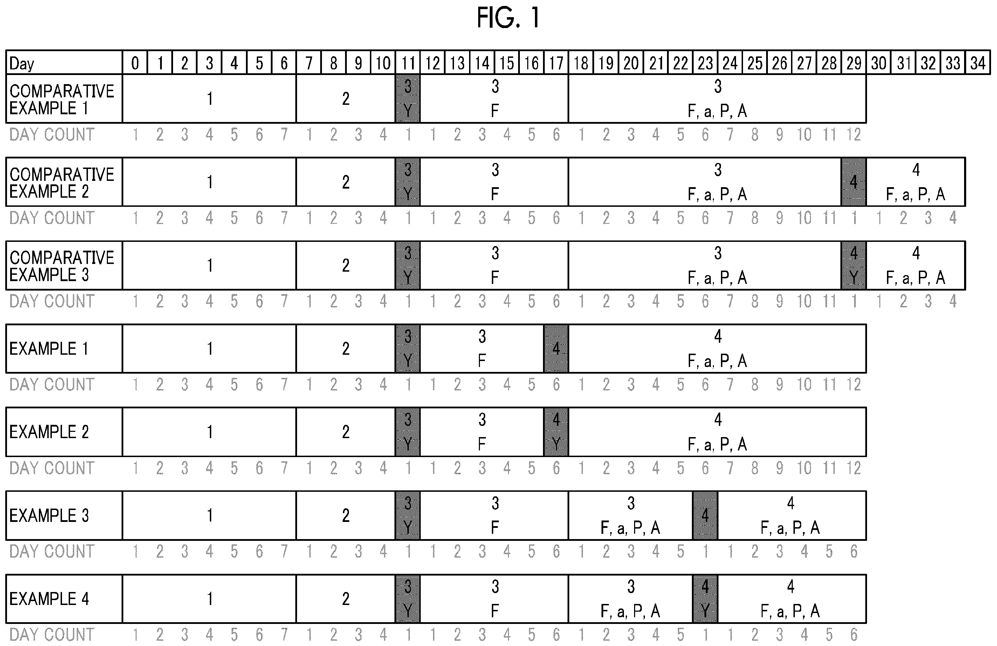

Fig. 1 illustrates an outline of the differentiation culture methods of Comparative Examples 1 to 3 and Examples 1 to 4.

Symbols and abbreviations in Fig.1 are as follows.
Culture condition 1:
   an RPMI 1640 medium containing Activin A and an RPMI 1640 medium containing BMP4, VEGF, FGF2, and EGF
Culture condition 2:
   DMEM/F12 containing 2% FBS, 1% Glutamax, and 250 ng/mL FGF2
Culture condition 3:
   DMEM/F12 containing 2% FBS, 1% Glutamax, 1% NEAA, 2% B27 supplement, 1% N2 supplement, 100 units/mL penicillin G, 100 µg/mL streptomycin, 20 ng/mL Epidermal growth factor (EGF)
Culture condition 4:
   Advanced DMEM/F12 (Thermo Fisher Scientific, Inc.) containing 20 ng/mL EGF
   Y: Y-27632
   F: Forskolin
   a: 5-aza-2'-deoxycytidine (5-aza-2'dc)
   P: PD98059
   A: A8301

In Comparative Example 1, cells were replated on day 11.

In Comparative Examples 2 and 3, cells were replated on day 11 and day 29.

In Examples 1 and 2, cells were replated on day 11 and day 17.

In Examples 3 and 4, cells were replated on day 11 and day 23.

### <Comparative Example 1>

Human iPS cells (FF-1) were cultured by culture in the presence of Activin A and culture in the presence of BMP4, VEGF, FGF2, and EGF for a total of 7 days (0th day to 7th day), and further cultured in DMEM/F12 containing 2% FBS, 1% Glutamax, and 250 ng/mL FGF2 for 4 days (7th day to 11th day) to induce differentiation into intestinal stem cells.

Thereafter, Y-27632 (a Rho-binding kinase inhibitor) was added so that the concentration thereof was 10 µmol/L, and the cells were treated at 37°C for 60 minutes in a CO₂ incubator. The treated cells were detached by treatment with accutase and plated on a cell culture well plate (surface area: 55 cm²) coated with Matrigel in advance.

Thererafter, culture was performed for 1 day (11th day to 12th day) in DMEM/F12 containing 2% FBS, 1% Glutamax, 1% NEAA, 2% B27 supplement, 1% N2 supplement, 100 units/mL penicillin G, 100 µg/mL streptomycin, 20 ng/mL epidermal growth factor (EGF), and 10 µmol/LY-27632, for 6 days (12th day to-18th day) in Advanced DMEM/F12 containing 2% FBS, 1% Glutamax, 1% NEAA, 2% B27 supplement, 1% N2 supplement, 100 units/mL penicillin G, 100 µg/mL streptomycin, 20 ng/mL epidermal growth factor (EGF), and 30 µmol/L Forskolin, and further for 12 days (18th day to 30th day) in the above culture medium to which 5 µmol/L 5-aza-2'dc, 20 µmol/L PD98059, 0.5 µmol/L A8301 were added, thereby obtaining an intestinal epithelial cell.

### <Comparative Example 2>

Human iPS cells (FF-1) were cultured for 11 days under the same conditions as in Comparative Example 1 to induce differentiation into intestinal stem cells. The cells were detached and plated under the same conditions as in Comparative Example 1.

Thereafter, under the same conditions as in Comparative Example 1, culture was performed for 1 day (11th day to 12th day), 6 days (12th day to 18th day), and further for 11 days (18th day to 29th day) after adding 5-aza-2'dc, PD98059, and A8301 as in Comparative Example 1. Then, cells were detached from the culture vessel with accutase and replated on the porous membrane (surface area: 0.33 cm²) on the cell culture insert.

After replating, culture was performed for 1 day (29th day to 30th day) in Advanced DMEM/F12 containing 20 ng/mL EGF and 4 days (30th day to 34th day) in Advanced DMEM/F12 containing 20 ng/mL EGF, 30 µmol/L Forskolin, 5 µmol/L 5-aza-2'dc, 20 µmol/L PD98059, and 0.5 µmol/L A8301, thereby obtaining intestinal epithelial cells.

### <Comparative Example 3>

On the day of replating (29th day), Y-27632 was added to a concentration of 10 µmol/L, and cells treated in a CO₂ incubator at 37°C for 60 minutes were detached with accutase, and replated on the porous membrane on the cell culture insert (surface area: 0.33 cm²). Y-27632 was added for 1 day until the culture medium exchange on the next day. Except for the changes described above, the cells were cultured in the same manner as in Comparative Example 2, thereby obtaining intestinal epithelial cells.

### <Example 1>

Human iPS cells (FF-1) were cultured for 11 days under the same conditions as in Comparative Example 1 to induce differentiation into intestinal stem cells. The cells were detached and plated under the same conditions as in Comparative Example 1.

Thereafter, under the same conditions as in Comparative Example 1, culture was performed for 1 day (11th day to 12th day), 5 days (12th day to 17th day). Then, cells were detached from the culture vessel with accutase and replated on the porous membrane (surface area: 0.33 cm²) on the cell culture insert.

After replating, culture was performed for 1 day (17th day to 18th day) in Advanced DMEM/F12 containing 20 ng/mL EGF and 12 days (18th day to 30th day) in Advanced DMEM/F12 containing 20 ng/mL EGF, 30 µmol/L Forskolin, 5 µmol/L 5-aza-2'dc, 20 µmol/L PD98059, and 0.5 µmol/L A8301, thereby obtaining intestinal epithelial cells.

### <Example 2>

On the day of replating (17th day), Y-27632 was added to a concentration of 10 µmol/L, and cells treated in a CO₂ incubator at 37°C for 60 minutes were detached with accutase and replated on the porous membrane on the cell culture insert (surface area: 0.33 cm²). Y-27632 was added for 1 day until the culture medium exchange on the next day. Except for the changes described above, the cells were cultured in the same manner as in Example 1, thereby obtaining intestinal epithelial cells.

### <Example 3>

Human iPS cells (FF-1) were cultured for 11 days under the same conditions as in Comparative Example 1 to induce differentiation into intestinal stem cells. The cells were detached and plated under the same conditions as in Comparative Example 1.

Thereafter, under the same conditions as in Comparative Example 1, culture was performed for 1 day (11th day to 12th day), 6 days (12th day to 18th day), and further for 5 days (18th day to 23rd day) after adding 5-aza-2'dc, PD98059, and A8301 as in Comparative Example 1. Then, cells were detached from the culture vessel with accutase and replated on the porous membrane (surface area: 0.33 cm²) on the cell culture insert.

After replating, culture was performed for 1 day (23rd day to 24th day) in Advanced DMEM/F12 containing 20 ng/mL EGF and 6 days (24th day to 30th day) in Advanced DMEM/F12 containing 20 ng/mL EGF, 30 µmol/L Forskolin, 5 µmol/L 5-aza-2'dc, 20 µmol/L PD98059, and 0.5 µmol/L A8301, thereby obtaining intestinal epithelial cells.

### <Example 4>

On the day of replating (23th day), Y-27632 was added to a concentration of 10 µmol/L, and cells treated in a CO₂ incubator at 37°C for 60 minutes were detached with accutase and replated on the porous membrane on the cell culture insert (surface area: 0.33 cm²). Y-27632 was added for 1 day until the culture medium exchange on the next day. Except for the changes described above, the cells were cultured in the same manner as in Example 1, thereby obtaining intestinal epithelial cells.

### <Test Example 1>

The CYP3A4 activity of the intestinal epithelial cells obtained in Comparative Examples 1 to 3 and Examples 1 to 4 was measured from the amount of a metabolite of midazolam. After the end of the induction of differentiation, cells were incubated in a culture medium containing 5 µmol/L midazolam at 37°C, and after 2 hours, the culture medium was sampled. The metabolic activity was calculated from the amount of 1-hydroxide midazolam in the culture medium, which was measured using a liquid chromatography-mass spectrometer (LC-MS/MS). After the end of the metabolism test, protein quantification was performed, and the metabolic activity was normalized with the amount of protein. The results are shown in Table 1.

As compared with Comparative Example 1, Examples 1, 2, and 4 showed comparable CYP3A4 activities. In Example 3, the CYP3A4 activity was slightly reduced, but the reduction was at a level that was not a problem in practical use.

### <Test Example 2>

Cells were plated in Transwell on the day of replating and cultured until the end date of the test, and then the barrier function (transepithelial electrical resistance: TEER) of the cells was measured in each test group. TEER was measured using EVOM² (registered trademark) Epithelial Volt/Ohm (TEER) Meter (WORLD PRECISION INSTRUMENTS). The operation followed the manual. The results are shown in Table 2.

Examples 1 to 4 showed a sufficient barrier function (200 Ω·cm² or more) similar to that of Comparative Example 1, but the barrier function was lost in Comparative Examples 2 and 3.

### <Test Example 3>

RNA was extracted, on the end date of test, from each of the intestinal epithelial cells of Comparative Examples 1 and Examples 1 to 4 in Test Example 2, in which the barrier function was maintained, using RNeasy (registered trademark) MiniKit (Qiagen). The operation was performed according to the attached manual. As the reverse transcription reaction, the synthesis of complementary DNA (cDNA) was performed using High capacity RNA-to-cDNA Kit (Applied Biosystems). The operation followed the attached manual.

Using the cDNA as a template, the real-time reverse transcription polymerase chain reaction (Real-Time RT-PCR) was carried out using TaqMan (registered trademark) Gene Expression Master Mix (Applied Biosystems). The operation followed the manual. Ribosome 18S was used as an internal control and the measurement results were normalized. The expression level of mRNA was estimated using probes of various genes. The results are shown in Table 3. The numerical values in Table 3 indicate the relative expression level in a case where the expression level in Adult Intestine is set to 100 for the small intestine marker and the relative expression level in a case where the expression level in Caco-2 is set to 100 for the liver marker.

The expressions of the small intestine markers Villin 1, CDX2, and ISX were confirmed in all of Comparative Example 1 and Examples 1 to 4. Further, in Examples 1 to 4, the expression levels of the liver markers albumin (ALB) and α-fetoprotein (AFP) were lower than those in Comparative Example 1. In particular, in Examples 3 and 4, the decrease in the expression levels of ALB and AFP was remarkable.

### <Test Example 4>

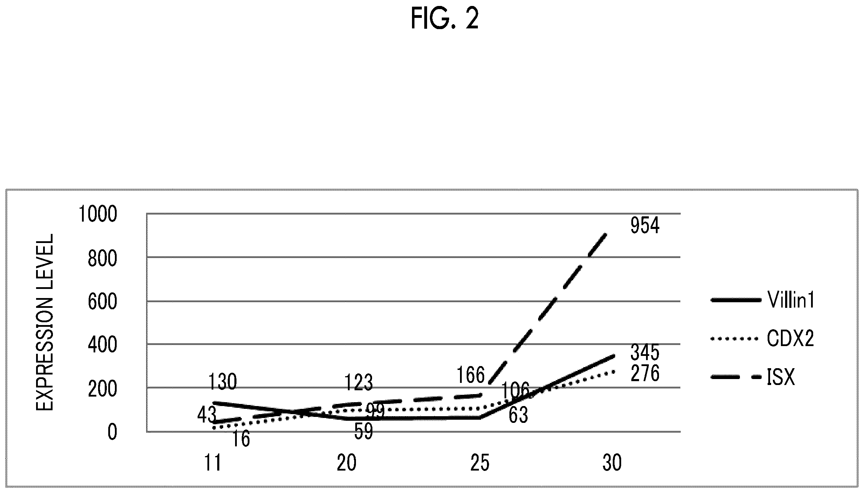

Human iPS cells (FF-1) were cultured and induced to differentiate under the same conditions as in Comparative Example 1. The culture and the induction of differentiation were started with dividing cells into 4 groups, and at the timing of Day 11, Day 20, Day 25, and Day 30, each group was subjected to the same test as in Test Example 3 to examine the expression of small intestine markers Villin1, CDX2, and ISX. The results are shown in Fig. 2. The numerical values in Fig. 2 indicate the relative expression levels in a case where the expression level in Adult Intestine is set to 100.

It has been found that the expression level of the small intestine marker increases drastically from Day 25 (that is, the differentiation into the intestinal epithelial cell progresses drastically). Considering these results together with the results of Test Example 2, it is desirable to perform the replating operation when the expression of the small intestine marker is the level around Day 25 in order not to adversely affect the barrier function of the intestinal epithelial cells.

**[Table 1]**

| CYP3A4 activity (pmol/2 hous/mg protein) | |
|---|---|
| Comparative Example 1 | 773 |
| Comparative Example 2 | 804 |
| Comparative Example 3 | 708 |
| Example 1 | 766 |
| Example 2 | 681 |
| Example 3 | 489 |
| Example 4 | 700 |

**[Table 2]**

| Barrier function (TEER: Ω·cm²) | |
|---|---|
| Comparative Example 1 | 674 |
| Comparative Example 2 | 0 |
| Comparative Example 3 | 0 |
| Example 1 | 732 |
| Example 2 | 537 |
| Example 3 | 988 |
| Example 4 | 816 |

**[Table 3]**

| mRNA expression level | | | | | |
|---|---|---|---|---|---|
| | Small intestine marker | | | Liver marker | |
| | Villin 1 | CDX2 | ISX | ALB | AFP |
| Comparative Example 1 | 341 | 414 | 981 | 3,780 | 3,730 |
| Example 1 | 334 | 378 | 925 | 1,759 | 3,078 |
| Example2 | 309 | 336 | 947 | 1,680 | 3,168 |
| Example3 | 148 | 359 | 788 | 20 | 168 |
| Example4 | 146 | 324 | 717 | 30 | 189 |
| Caco-2 | 555 | 607 | 105 | *100* | *100* |
| Adult Intestine | 100 | 100 | 100 | 0 | 0 |
| Adult Liver | 9 | 0 | 0 | 36,298 | 0 |

## Claims

1. A method for producing an intestinal epithelial cell, comprising:
a step 1-1 of differentiating a pluripotent stem cell into an endodermal cell,
a step 1-2 of differentiating the endodermal cell into an intestinal stem cell;
a step of treating the intestinal stem cell with ROCK inhibitor;
a step of replating the treated intestinal stem cell; and
a step 2 of differentiating the intestinal stem cell obtained in the step 1 into an intestinal epithelial cell in a presence of EGF, a cAMP activator and one or more selected from the group consisting of a MEK1 inhibitor, a DNA methylation inhibitor, and a TGFβ receptor inhibitor,
wherein the period of step 1-1 is 2 days to 7 days, the period of step 1-2 is 3 days to 7 days, and the period of step 2 is 7 days to 40 days, and
wherein during the step 2, a cell under differentiation is replated one or more times at the following timings while maintaining the barrier function;
(a) a timing after 4th day after a start of the step 2 and 5 days or more before an end of the step 2,
wherein the cAMP activator is Forskolin, indomethacin, NKH477 (colforsin daropate), pertussis toxin, cholera toxin, PACAP-27, PACAP-38, or SKF8382.

2. The method according to claim 1,
wherein during the step 2, the cell under differentiation is replated one or more times at the following timings;
(a) a timing after 10th day after the start of the step 2 and before 5 days or more from an end of the step 2.

3. The method according to claim 1 or 2,
wherein the step 2 includes a step of differentiating the intestinal stem cell obtained in the step 1 into the intestinal epithelial cell in a presence of a MEK1 inhibitor, a DNA methylation inhibitor, a TGFβ receptor inhibitor, EGF, and a cAMP activator.

4. The method according to any one of claims 1 to 3,
wherein in the step 2, the cell is replated on a porous membrane.

5. The method according to any one of claims 1 to 4,
wherein the steps 1-1 and 1-2 are performed on a culture plate having a surface area of 30 cm² or more.

6. The method according to any one of claims 1 to 5,
wherein culture after the replating in the step 2 is performed on a culture plate having a surface area of 3 cm² or less per well.

## Patentansprüche

1. Verfahren zum Produzieren einer Darmepithelzelle, umfassend:
einen Schritt 1-1 des Differenzierens einer pluripotenten Stammzelle in eine endodermale Zelle;
einen Schritt 1-2 des Differenzierens der endodermalen Zelle in eine Darmstammzelle;
einen Schritt des Behandelns der Darmstammzelle mit ROCK-Inhibitor;
einen Schritt des erneuten Plattierens der behandelten Darmstammzelle; und
einen Schritt 2 des Differenzierens der bei dem Schritt 1 erhaltenen Darmstammzelle in eine Darmepithelzelle in einer Gegenwart von EGF, einem cAMP-Aktivator und einem oder mehreren, die aus der Gruppe, die aus einem MEK1-Inhibitor, einem DNA-Methylierungsinhibitor und einem TGFβ-Rezeptor-Inhibitor besteht, ausgewählt werden,
wobei der Zeitraum von Schritt 1-1 2 Tage bis 7 Tage beträgt, der Zeitraum von Schritt 1-2 3 Tage bis 7 Tage beträgt und der Zeitraum von Schritt 2 7 Tage bis 40 Tage beträgt, und
wobei während des Schritts 2 eine Zelle, die unter Differenzierung steht, zu den folgenden Zeitpunkten einmal oder mehrmals erneut plattiert wird, während die Barrierefunktion aufrechterhalten wird;
(a) einem Zeitpunkt nach 4. Tag nach einem Beginn des Schrittes 2 und 5 Tage oder mehr vor einem Ende des Schrittes 2,
wobei der cAMP-Aktivator Forskolin, Indomethacin, NKH477 (Colforsin-Daropate), Pertussistoxin, Cholera-Toxin, PACAP-27, PACAP-38 oder SKF8382 ist.

2. Verfahren nach Anspruch 1,
wobei während des Schritts 2 die Zelle unter Differenzierung einmal oder mehrmals zu den folgenden Zeitpunkten erneut plattiert wird;
(a) einem Zeitpunkt nach 10. Tag nach dem Beginn des Schritts 2 und vor 5 Tagen oder mehr ab einem Ende des Schritts 2.

3. Verfahren nach Anspruch 1 oder 2,
wobei der Schritt 2 einen Schritt des Differenzierens der bei dem Schritt 1 erhaltenen Darmstammzelle in die Darmepithelzelle in einer Gegenwart eines MEK1 -Inhibitors, eines DNA-Methylierungsinhibitors, eines TGFβ-Rezeptor-Inhibitors, EGF und eines cAMP-Aktivators enthält.

4. Verfahren nach einem der Ansprüche 1 bis 3,
wobei bei dem Schritt 2 die Zelle auf einer porösen Membran erneut plattiert wird.

5. Verfahren nach einem der Ansprüche 1 bis 4,
wobei die Schritte 1-1 und 1-2 an einer Kulturplatte mit einer Oberflächenfläche von 30 cm² oder mehr durchgeführt werden.

6. Verfahren nach einem der Ansprüche 1 bis 5,
wobei Kultur nach dem erneuten Plattieren bei dem Schritt 2 auf einer Kulturplatte mit einer Oberflächenfläche von 3 cm² oder weniger pro Wanne durchgeführt wird.

## Revendications

1. Procédé pour produire une cellule épithéliale intestinale comprenant :
une étape 1-1 de différenciation d'une cellule souche pluripotente en une cellule endodermique ;
une étape 1-2 de différenciation de la cellule endodermique en une cellule souche intestinale ;
une étape de traitement de la cellule souche intestinale avec un inhibiteur de ROCK ;
une étape de replacage de la cellule souche intestinale traitée ; et
une étape 2 de différenciation de la cellule souche intestinale obtenue à l'étape 1 en une cellule épithéliale intestinale en présence d'EGF, d'un activateur de cAMP et d'un ou plusieurs éléments sélectionnés parmi le groupe constitué d'un inhibiteur de MEK1, d'un inhibiteur de méthylation de l'ADN et d'un inhibiteur de récepteur de TGFβ,
dans lequel la période de l'étape 1-1 est de 2 jours à 7 jours, la période de l'étape 1-2 est de 3 jours à 7 jours, et la période de l'étape 2 est de 7 jours à 40 jours, et
dans lequel pendant l'étape 2, une cellule en cours de différenciation est replaquée une ou plusieurs fois aux moments suivants tout en maintenant la fonction barrière ;
(a) un moment après 4ème jour après un début de l'étape 2 et 5 jours ou plus avant une fin de l'étape 2,
dans lequel l'activateur de cAMP est Forskoline, indométhacine, NKH477 (colforsin daropate), toxine de coqueluche, toxine cholérique, PACAP-27, PACAP-38 ou SKF8382.

2. Procédé selon la revendication 1,
dans lequel pendant l'étape 2, la cellule en cours de différenciation est replaquée une ou plusieurs fois aux moments suivants ;
(a) un moment après 10e jour après le début de l'étape 2 et avant 5 jours ou plus à partir d'une fin de l'étape 2.

3. Procédé selon la revendication 1 ou la revendication 2,
dans lequel l'étape 2 inclut une étape de différenciation de la cellule souche intestinale obtenue à l'étape 1 en la cellule épithéliale intestinale en présence d'un inhibiteur de MEK1, d'un inhibiteur de méthylation de l'ADN, d'un inhibiteur de récepteur de TGFβ, de EGF et d'un activateur de cAMP.

4. Procédé selon l'une quelconque des revendications 1 à 3,
dans lequel à l'étape 2, la cellule est replaquée sur une membrane poreuse.

5. Procédé selon l'une quelconque des revendications 1 à 4,
dans lequel les étapes 1-1 et 1-2 sont effectuées sur une plaque de culture ayant une surface de 30 cm² ou plus.

6. Procédé selon l'une quelconque des revendications 1 à 5,
dans lequel la culture après le replacage à l'étape 2 est effectuée sur une plaque de culture ayant une surface de 3 cm² ou moins par puits.
